# FASCICULE DE BREVET EUROPEEN

(11) **EP 2 911 588 B1**
(45) Date de publication et mention de la délivrance du brevet: **13.10.2021**
(21) Numéro de dépôt: 13780151.0
(22) Date de dépôt: 24.10.2013
(51) Int. Cl.: A61B 7/00, A61B 5/00

(54) **DÉTECTEUR DE SON ÉTANCHE DOTÉ DE DEUX CAPTEURS POUR LA MESURE DU SON ET DE LA PRESSION STATIQUE**
SCHALLDICHTER DETEKTOR MIT ZWEI SENSOREN ZUR MESSUNG VON SCHALL- UND STATISCHEM DRUCK
SEALED SOUND DETECTOR PROVIDED WITH TWO SENSORS FOR MEASURING SOUND AND STATIC PRESSURE

(30) Priorité: 29.10.2012 FR 1260312
(43) Date de publication de la demande: 02.09.2015
(73) Titulaire: Controle Instrumentation Et Diagnostic Electroniques SA (Cidelec), 49130 Sainte-Gemmes-sur-Loire (FR)
(72) Inventeur: FREYCENON, Cédric, F-49320 Vauchretien (FR); MONTARON, Christian, F-49080 Bouchemaine (FR)
(74) Mandataire: Vidon Brevets & Stratégie
(86) Numéro de dépôt international: PCT/EP2013/072232
(87) Numéro de publication internationale: WO 2014/067837

(56) Documents cités:
- US-A- 5 467 775
- US-A1- 2008 154 144
- US-B2- 8 116 841

## Description

### 1. Domaine de l'invention

L'invention concerne un détecteur acoustique étanche capable de capter des signaux très basse fréquence et de mesurer des pressions statiques. De tels détecteurs peuvent être utilisés dans le domaine médical, en particulier pour déterminer les caractéristiques respiratoires d'un patient pendant son sommeil et détecter la présence d'éventuelles apnées.

### 2. Art antérieur

Des individus peuvent être atteints d'une pathologie appelée « syndrome d'apnée du sommeil » qui consiste à arrêter la respiration pendant le sommeil. Ces absences de cycles respiratoires, d'une durée supérieure à 10 secondes, nuisent à la qualité du sommeil et provoquent une diminution du taux en oxygène du sang artériel. La diminution en oxygène détériore peu à peu les fonctions cérébrales et cardiaques, et la multiplication de micro-réveils empêche le cerveau d'entrer dans la phase de sommeil profond. Les individus atteints par cette pathologie ont un risque plus important d'accidents cardiovasculaires et de somnolence diurne responsable notamment d'accidents de la route ou du travail. Il importe donc de disposer d'un appareillage médical capable de détecter ce genre de pathologie chez des patients, afin de les informer de leur état et de les soigner.

Cet appareillage consiste principalement à enregistrer le comportement respiratoire du patient pendant son sommeil et à analyser les mesures afin d'identifier d'éventuels événements pathologiques tels que les apnées et les hypopnées.

Différentes techniques peuvent être mises en œuvre pour mesurer la ventilation du patient. Une première technique consiste à utiliser un masque buco-nasal et à enregistrer les mouvements d'air. Cependant, beaucoup de patients ne supportent pas le port d'un tel masque et sa simple présence peut en soi perturber le sommeil. Une seconde technique utilise une lunette nasale et une thermistance, placées sous les narines du patient et au niveau de la bouche, pour obtenir des informations sur la présence ou l'absence de débit de ventilation. Cette méthode est moins sensible que la première et pose de nombreux problèmes quant au positionnement et à la fixation des capteurs.

Un autre appareillage consiste à mesurer les élongations thoraciques et abdominales à l'aide de sangles inductives. Cet appareillage doit cependant être complété avec des capteurs de flux d'air au niveau du nez et de la bouche. De plus cet appareillage nécessite un étalonnage des sangles qui est long et approximatif. Enfin, certains patients ne supportent pas la contrainte d'avoir une sangle autour de la poitrine.

Un autre appareillage consiste à placer un capteur de pression sur la base du cou du patient pour détecter les bruits résultants de la respiration. La demande de brevet FR 2 664 487 déposée par l'Université d'Angers décrit l'utilisation de trois capteurs de pression destinés à détecter des signaux dans des gammes de fréquences différentes. Une unité de traitement analyse les signaux générés par les capteurs de pression (son débit, son ronflement, pression susternale) et détermine les paramètres de la respiration du patient. En pratique, ce capteur permet la détection des apnées (présence ou absence de débit) et la caractérisation de celles-ci avec la pression susternale (origine obstructive, centrale, ou mixte). Cependant la technique de mesure, par sa sensibilité faible sur la pression statique, peut induire des problèmes d'interprétations (en particulier sur les apnées mixtes et en pédiatrie à cause du décalage du signal), D'autre part, la manière de protéger les capteurs de pression contre les agressions chimiques (sueur du patient) n'est pas décrite.

La demande de brevet US 2008/0154144, publiée le 26 juin 2008 décrit un système d'auscultation comprenant un transducteur générant un signal acoustique à un endroit précis du patient, et un capteur permettant de détecter un signal acoustique atténué en un autre endroit du patient. Ce détecteur ne possède pas d'éléments de protection contre d'éventuelles agressions chimiques.

Il existe donc un réel besoin d'un détecteur de son apte à mesurer des sons dans une large gamme de fréquence et utilisable dans un milieu présentant des agressions chimiques.

### 3. Objectifs de l'invention

Les appareillages actuels présentent soit un inconfort d'utilisation, soit un manque de sensibilité empêchant de détecter des mouvements respiratoires et de déterminer le type d'apnée.

L'invention apporte une solution qui ne présente pas les inconvénients décrits plus haut, en proposant un appareil ayant une forme qui présente très peu d'inconfort pour le patient, et offre une grande sensibilité permettant notamment de mesurer les sons pour détecter les arrêts respiratoires, et de mesurer la pression statique pour permettre la classification des apnées.

### 4. Exposé de l'invention

En vue de résoudre au moins les problèmes mentionnés précédemment, la présente invention propose un détecteur de son comprenant un boitier doté d'une première ouverture sur l'extérieur pour la mesure de la pression statique et du son. Le détecteur inclut un microphone délivrant des signaux représentatifs du son entrant par la première ouverture. Le boitier inclut également un capteur de pression délivrant des signaux représentatifs d'une pression statique et d'un son d'une fréquence inférieure aux fréquences captées par le microphone. De cette manière, le même détecteur peut à la fois mesurer une pression statique et les caractéristiques des sons dus à la respiration d'un patient, évitant ainsi l'utilisation de sangles périthoraciques ou péri-abdominales et simplifiant le traitement.

Pour assurer une étanchéité et permettre l'utilisation du détecteur dans le domaine médical, le microphone détecte le son entrant par la première ouverture à travers une membrane étanche en polyimide. De cette manière, la partie inférieure du détecteur de son est parfaitement étanche, il peut être utilisé dans le domaine médical et notamment être appliqué directement sur l'épiderme des patients.

Selon un autre mode de réalisation, la membrane en polyimide est insérée entre deux couches de substrat d'un circuit imprimé. Les deux couches de substrat possèdent un trou qui est fermé par la membrane et qui débouche vers le microphone et vers la première ouverture. De cette manière, la membrane est solidaire du circuit imprimé et assure une bonne étanchéité vers le milieu extérieur.

Selon un autre mode de réalisation, le microphone, ou le microphone et le capteur de pression, capte des caractéristiques physiques présentes dans une chambre acoustique de forme conique, la partie la plus large étant dirigée vers la première ouverture. De cette manière, le volume de la chambre fermée par la membrane est plus petit et communique mieux les caractéristiques physiques aux différents capteurs.

Selon un autre mode de réalisation le détecteur comporte un capteur de position et délivre des signaux représentatifs de la position du détecteur dans l'espace. De cette manière, lorsque le détecteur de son est appliqué sur un patient, il peut indiquer la position dudit patient pendant son sommeil.

### 5. Liste des figures

D'autres caractéristiques et avantages de l'invention apparaîtront plus clairement à la lecture de la description suivante d'un mode de réalisation particulier, donné à titre de simple exemple illustratif et non-limitatif, et des dessins annexés, parmi lesquels :
- La figure 1 présente un individu dans une position de sommeil avec un détecteur de son relié à une unité de traitement,
- La figure 2 présente un synoptique de la structure d'un détecteur de son selon un exemple préféré de réalisation,
- la figure 3 présente un schéma en coupe du circuit imprimé comprenant les principaux composants électroniques,
- la figure 4 présente un schéma du circuit imprimé inséré dans son boitier mécanique, et
- la figure 5 montre un exemple de schéma électronique des composants du détecteur selon un exemple de réalisation.

### 6. Description d'un mode de réalisation de l'invention

### 6.1 Principe général

Le principe général de l'invention repose sur un détecteur de son doté d'un capteur de pression pour la mesure des signaux acoustiques de très basse fréquence et la mesure d'une pression statique, et d'un microphone pour capter les signaux acoustiques de moyenne fréquence. Les signaux sont traités par des circuits électroniques destinés à les amplifier avant de les transmettre à une unité de traitement où ils seront analysés. Le microphone débouche sur une chambre acoustique fermée par une membrane en polyimide directement intégrée au circuit imprimé et supportant des circuits électroniques, assurant ainsi l'étanchéité avec le milieu extérieur.

Dans des applications médicales, ledit détecteur est notamment placé sur la poitrine ou le cou d'un individu pour détecter ses mouvements respiratoires, et permet de déterminer des phases d'apnée pendant le sommeil.

L'utilisation d'une membrane dans une matière telle que le polyimide, peut être utile partout où il convient de recueillir des sons avec une grande sensibilité, dans un espace réduit, tout en protégeant le microphone des agressions chimiques extérieures. La réalisation de la membrane dans le circuit imprimé simplifie l'intégration de l'ensemble et permet une miniaturisation importante.

### 6.2 Description d'un mode de réalisation

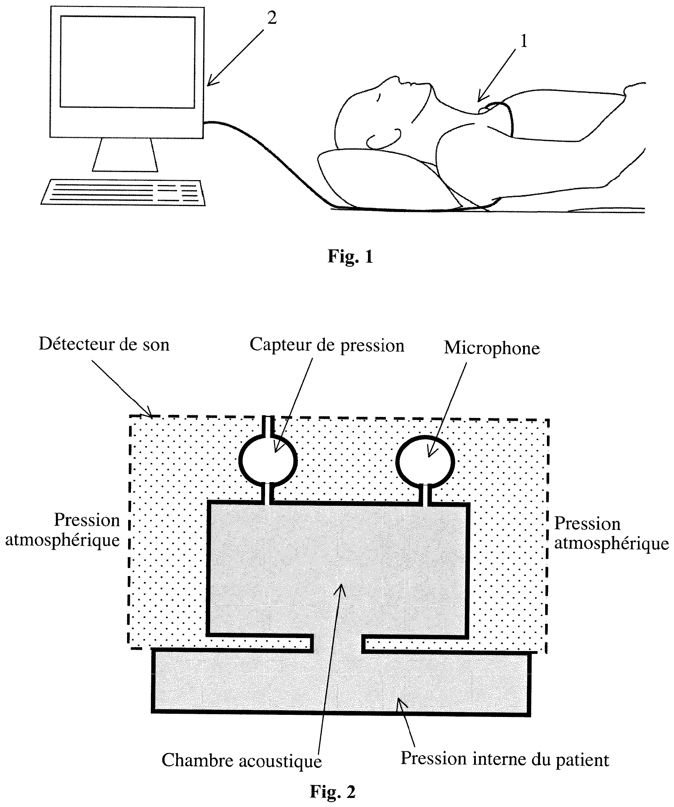

La **FIG.1** présente un individu dans une position de sommeil avec un détecteur de son relié à une unité de traitement. Le détecteur de son 1 est placé à la base du cou de l'individu ou sur son thorax, et maintenu par un adhésif médical. Selon un mode préféré de réalisation, le détecteur 1 dispose d'un câble relié à une unité de traitement 2 qui analyse les signaux sonores émis par l'individu et détermine ses caractéristiques respiratoires : inspiration, expiration, mouvement de la glotte, etc.

D'un point de vue mécanique, le voisinage du corps humain peut être schématisé par une paroi élastique, l'épiderme, séparant le milieu extérieur, à savoir l'air à la pression atmosphérique, du milieu intérieur, à savoir la pression dans le médiastin. Toute modification locale de la pression interne va se traduire par une déformation locale de la paroi au niveau de l'orifice de la chambre des capteurs suivant les lois de la mécanique. De plus, les flux d'air se déplaçant dans la gorge et la trachée artère génèrent des sons qui se propagent à travers les différents tissus de l'individu et sont parfaitement audibles sur l'épiderme

La **FIG.2** présente un synoptique de la structure d'un détecteur de son selon un exemple préféré de réalisation.

L'environnement du détecteur 1 est soumis à la pression atmosphérique. La partie inférieure du détecteur est constituée d'une chambre acoustique débouchant sur un évent qui vient se poser sur l'épiderme du patient. Le bord de l'évent étant en contact avec l'épiderme, la chambre acoustique constitue une cavité fermée. De ce fait, les variations de pression dans la chambre acoustique dépendent des sons et de la pression provoqués par la respiration et les mouvements respiratoires. Un microphone classique n'est pas capable de détecter des sons très basse fréquence et ne peut en aucun cas détecter une pression statique. Les signaux que l'on souhaite acquérir avec le détecteur objet de la présente invention couvrent une gamme de fréquences qui s'étend d'une pression statique (fréquence nulle) à des sons d'une fréquence de plusieurs milliers d'Hertz. Pour mesurer une telle gamme de fréquence acoustique, l'invention prévoit d'adjoindre deux capteurs : un microphone conçu pour détecter des sons de 100 Hz à 10000 Hz, par exemple, et un capteur de pression conçu pour mesurer une pression statique et détecter de faibles mouvements acoustiques de fréquence nulle à 1000 Hz. L'adjonction d'un capteur de pression permet d'augmenter la bande passante dans la gamme des très basses fréquences et de détecter des pressions statiques. De cette façon, le détecteur 1 produit des signaux électriques plus riches en information. Selon un mode particulier de réalisation, le capteur de pression est de type différentiel et possède deux évents pour mesurer la différence de pression entre la pression atmosphérique et la pression de l'épiderme du patient. Le microphone détecte les sons à l'intérieur de la chambre acoustique, ces sons étant transmis par l'épiderme du patient.

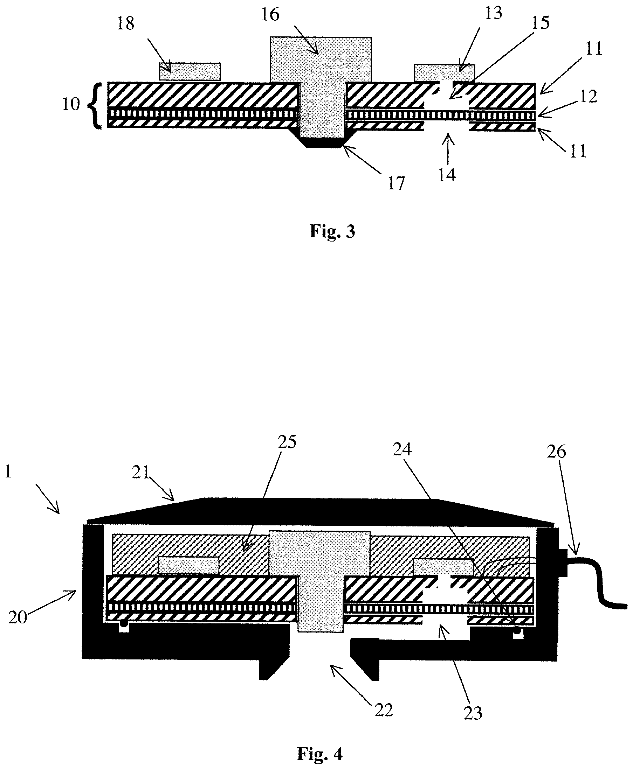

La **FIG.3** présente un schéma en coupe du circuit imprimé comprenant les principaux composants équipant le détecteur.

Le circuit imprimé 10 de forme circulaire se compose de deux couches 11 de substrat intercalant une membrane en polyimide 12 le tout étant maintenu par collage. Le polyimide a la particularité d'être parfaitement étanche et d'être suffisamment souple pour transmettre les sons sur une large gamme de fréquence, sans les altérer. Si le circuit imprimé possède des pistes sur les deux faces, la membrane 12 et les deux couches 11 de substrat sont percées par des trous métallisés, bouchés par la soudure.

Un microphone 13 est soudé sur une face du circuit imprimé, les contacts électriques sont reliés aux pistes tracées sur cette face du circuit. La partie détection du microphone est placée sur un premier trou 14 creusé à travers les deux couches 11 de substrat et avantageusement disposée en périphérie du circuit imprimé. La membrane 12 n'est pas percée au niveau du premier trou, ménageant ainsi une première chambre acoustique fermée 15. De cette façon, les vibrations sonores peuvent passer à travers la membrane, mais la chambre 15 est étanche et aucune impureté ne peut atteindre le microphone. Un second trou est pratiqué dans le circuit imprimé pour placer un capteur de pression 16. Le capteur de pression 16 est avantageusement un capteur protégé par un gel. Le capteur 16 est également relié électriquement aux pistes du circuit imprimé.

De façon optionnelle, le détecteur comporte un détecteur de position 18 qui permet de déterminer la position du patient. La **FIG. 3** ne montre pas les autres composants électroniques (résistances, condensateur, amplificateur opérationnel, ...) présents sur le circuit imprimé.

La **FIG.4** présente le boitier mécanique avec les éléments assurant l'étanchéité et la protection des principaux composants et capteurs du détecteur 1. Le circuit imprimé et ces composants sont logés dans un boitier circulaire 20 fermé par un couvercle 21 constituant la partie supérieure du détecteur 1. Le couvercle 21 est maintenu par des clips au boitier, ou selon une variante de réalisation, le couvercle 21 est vissé sur le boitier 20. Le boitier circulaire 20 possède une ouverture centrale 22 en forme d'évent dans sa partie inférieure. L'évent débouche sur une seconde chambre acoustique 23 formée par le fond et le coté cylindrique du boitier, et par la face inférieure du circuit imprimé 11 et la membrane 12. La seconde chambre acoustique constitue un volume fermé lorsque l'évent du boitier est en contact avec l'épiderme d'un individu soumis à un examen. La fixation du détecteur 1 sur l'épiderme de l'individu s'effectue au moyen d'une collerette adhésive double face qui entoure l'ouverture 22 sur la face inférieure du détecteur 1. La collerette adhésive assure l'étanchéité de la chambre acoustique 23 et permet l'apparition des variations de pression liées aux mouvements de l'épiderme. La transpiration ou la poussière qui pourraient pénétrer dans la chambre acoustique 23 par l'ouverture 22, sont bloqués par la membrane 12 et ne peuvent endommager le microphone 13. Un joint silicone circulaire 24 est placé entre la périphérie de la face inférieure du circuit imprimé et le fond du boitier pour assurer l'étanchéité de la chambre acoustique 23. Il est aussi possible de supprimer ce joint 24 en collant directement le substrat 11, constituant la partie inférieure du circuit imprimé, au fond du boitier 20. Il faut noter que l'étanchéité entre le boitier 20 et le couvercle n'est pas nécessaire et n'est pas non plus recherchée. En effet, le capteur de pression 16 enregistre des pressions différentielles entre la chambre acoustique 23 et la pression extérieure. Un des évents de mesure du capteur de pression 16 débouche dans la chambre acoustique et l'autre évent débouche à l'intérieur du boitier, sous le couvercle. De façon optionnelle, les circuits et capteurs 13, 16 et 18 placés sur le circuit imprimé 10 sont recouverts d'un gel en silicone 25 pour assurer une meilleure protection et une meilleure tenue mécanique. On prendra soin de ne pas recouvrir de gel 25 l'évent du capteur de pression 16 débouchant à l'intérieur du boitier 20.

Selon un mode préféré de réalisation, le détecteur de son 1 est muni d'un câble 26 de connexion permettant de transmettre les signaux représentatifs d'une pression statique et des sons captés par le microphone vers une unité de traitement 2 capable de les analyser. Le câble de connexion est de type multibrin, il apporte l'alimentation électrique aux différents circuits électroniques du détecteur 1 et transmet vers l'unité de traitement 2 les signaux électriques en provenance de chacun des capteurs 13, 16 et 18. Une variante de la présente invention consiste à munir le boîtier d'une source autonome d'énergie (pile ou batterie) et à transmettre les signaux représentatifs de la respiration de l'individu sous examen par une liaison sans contact (onde radio, micro-ondes, rayonnement infra-rouge, etc).

Le prototype de détecteur de son 1 réalisé possède un diamètre extérieur de 22 millimètres pour une épaisseur totale de 10 millimètres. La membrane en polyimide a une épaisseur de 25 microns, et chaque couche de substrat 11 a une épaisseur totale 570 microns. En prenant en compte les épaisseurs de colle, l'épaisseur du circuit imprimé est de 1300 microns. En variante, les épaisseurs des couches de substrat peuvent être différentes, et le nombre de couches peut être supérieur à deux. Le diamètre du premier trou débouchant sous le microphone est de 0.6 millimètre et va s'élargissant jusqu'à un diamètre de 10 mm procurant une forme conique à la première chambre acoustique 15. Cette forme permet d'adapter la dimension relativement petite (0.6 mm) de l'ouverture du microphone et celle de la surface de membrane qui doit être la plus grande possible pour une meilleure propagation des sons et de la pression. D'autre part, plus la chambre est petite, plus les mouvements de l'épiderme provoqueront des variations importantes de pressions, mouvements détectés par le capteur de pression 16.

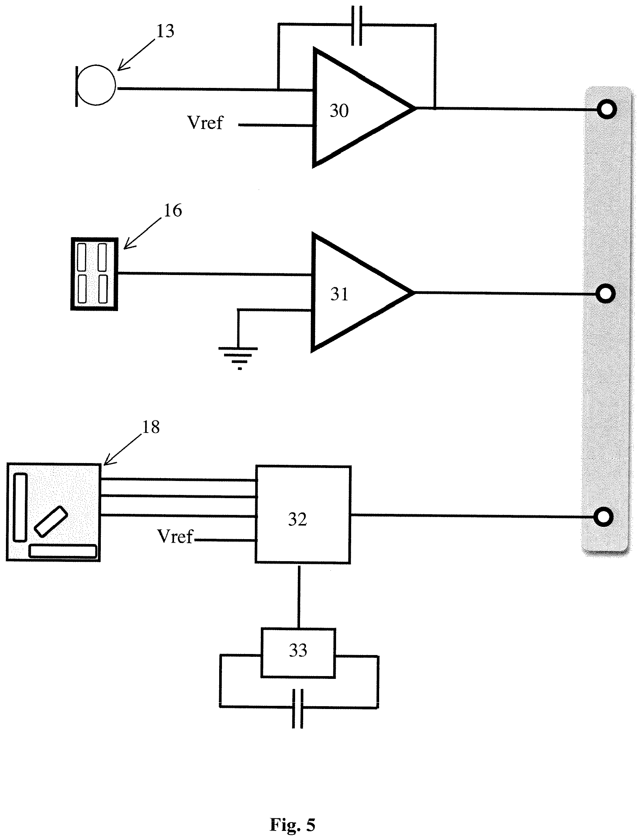

La **FIG.5** présente un exemple de schéma électronique des composants du détecteur 1 selon un exemple de réalisation.

Trois circuits électroniques ayant en commun la même alimentation sont disposés sur le circuit imprimé. Un premier circuit comprend le microphone 13 relié à un circuit d'amplification 30. Un second circuit comprend le capteur de pression 16 relié à un second circuit d'amplification 31. Les signaux ainsi amplifiés sont transmis par un câble vers une unité de traitement. Les signaux de ces deux capteurs sont traités simultanément afin de déterminer les phases de respiration et celles d'absence de respiration, ainsi que la présence ou l'absence de variation de pression intra pulmonaire, signe d'efforts respiratoires. On rappelle qu'une apnée est une absence de respiration pendant au moins 10 secondes.

Un troisième circuit comprend le détecteur de position 18. Ce détecteur fournit une mesure de l'accélération selon trois axes orthogonaux X, Y, Z permettant, lorsque le détecteur est immobile, de fournir la mesure de gravité selon les trois axes et donc de déterminer la position du détecteur dans l'espace. Le détecteur étant placé sur le cou du patient, les mouvements du patient pendant son sommeil sont communiqués au détecteur de position 18. De sorte que les informations fournies par ce détecteur permettent de déterminer la position du patient. Les signaux de sortie sont transmis par des fils regroupés dans un câble multibrin présent en partie droite de la figure 5. Une variante consiste à disposer d'un multiplexeur pour transmettre les signaux séquentiellement sur le même fil de communication.

Une nomenclature des principaux circuits électroniques utilisés dans le prototype de détecteur de son 1 est donnée en annexe à titre purement indicatif. Cette liste constitue un exemple de réalisation et ne limite en rien la portée de l'invention.

L'unité de traitement enregistre les informations au cours d'une longue période de sommeil et peut les restituer sous la forme d'un chronogramme affiché sur un écran ou imprimé sur une feuille de papier. L'analyse conjointe des variations de pression statique et des sons permet de déterminer les cycles inspiratoires et expiratoires. Selon l'art antérieur, cela permet d'analyser les variations des résistances des voies aériennes au cours du cycle respiratoire.

Dans la variante consistant à disposer d'un module de communication sans contact, les signaux provenant des différents capteurs sont numérisés par un convertisseur AD à plusieurs entrées. Une unité de multiplexage sélectionne l'entrée du convertisseur AD et reçoit la données numérique sur un bus série afin de l'envoyer vers une unité de transmission sans contact. Des valeurs binaires d'en-tête permettent d'identifier les valeurs numériques transmises selon le type de capteur. Selon cette variante, il est inutile de prévoir une liaison bidirectionnelle. Le détecteur est avantageusement muni d'un bouton poussoir pour afficher à l'aide d'un voyant lumineux l'état de l'alimentation autonome afin d'inciter l'utilisateur à changer la pile ou à recharger la batterie.

La présente invention concerne un détecteur de son décrit par les revendications jointes qui peut être utilisé dans le domaine médical, ou d'autres domaines où il est utile de mesurer une large gamme de caractéristiques physiques et où des agressions chimiques nécessitent une bonne étanchéité.

**ANNEXE**

| Référence (figures) | Type de circuit | fabriquant | Référence |
|---|---|---|---|
| 13 | microphone | Knowles Accoustics | SPU0409LE5H |
| 16 | Capteur de pression | Nova Sensor | NPC-100 |
| 18, 32 | Capteur de position | ST Micro-Electronique | LIS302SG |
| 30 | Ampli opérationnel | Analog Device | AD8603AUJ |
| 31 | Ampli opérationnel | Maxim | MAX4194ESA |
| 33 | Générateur d'horloge | Linear Technologie | LTC6991 |

## Revendications

1. Détecteur de son (1) comprenant un boitier (20) doté d'une première ouverture (22) sur l'extérieur pour la mesure de la pression statique et du son, ledit détecteur incluant un microphone (13) délivrant des signaux représentatifs du son entrant par la première ouverture (22), ledit boitier incluant également un capteur de pression (16) délivrant des signaux représentatifs d'une pression statique et d'un son d'une fréquence inférieure aux fréquences captées par le microphone (13) ; où ledit microphone détecte le son entrant par la première ouverture (22) à travers une membrane ; **caractérisé en ce que** ladite membrane est étanche (12) et en polyimide, et **en ce que** ladite membrane (12) est insérée entre deux couches (11) de substrat d'un circuit imprimé (10) supportant des circuits électroniques intégrés dans le détecteur de son (1).

2. Détecteur de son (1) selon la revendication 1, **caractérisé en ce que** les deux couches de substrat ont un trou fermé par ladite membrane (12) et débouchant vers le microphone (13) et vers la première ouverture (22).

3. Détecteur de son (1) selon l'une quelconque des revendications 1 ou 2, **caractérisé en ce que** le microphone (13), ou le microphone (13) et le capteur de pression (16), captent des caractéristiques physiques présentes dans une chambre acoustique (15) de forme conique, la partie la plus large étant dirigée vers la première ouverture (22).

4. Détecteur de son (1) selon l'une quelconque des revendications précédentes, **caractérisé en ce qu'**il comporte un capteur de position et **en ce que** ledit détecteur de son délivre des signaux représentatifs de la position du détecteur dans l'espace.

## Patentansprüche

1. Schalldetektor (1), welcher ein Gehäuse (20) umfasst, das mit einer ersten Öffnung (22) nach außen zur Messung des statischen Drucks und des Schalls ausgestattet ist, wobei der Detektor ein Mikrofon (13) enthält, das Signale liefert, die für den durch die erste Öffnung (22) eintretenden Schall repräsentativ sind, wobei das Gehäuse außerdem einen Drucksensor (16) enthält, der Signale liefert, die für einen statischen Druck und einen Schall mit einer Frequenz repräsentativ sind, die niedriger als die von dem Mikrofon (13) aufgenommenen Frequenzen ist; wobei das Mikrofon den durch die erste Öffnung (22) eintretenden Schall durch die Membran hindurch detektiert;
**dadurch gekennzeichnet, dass** die Membran (12) dicht und aus Polyimid ist, und dadurch, dass die Membran (12) zwischen zwei Substratschichten (11) einer Leiterplatte (10) eingesetzt ist, die in den Schalldetektor (1) integrierte elektronische Schaltungen trägt.

2. Schalldetektor (1) nach Anspruch 1, **dadurch gekennzeichnet, dass** die zwei Substratschichten ein Loch aufweisen, das durch die Membran (12) verschlossen ist und zum Mikrofon (13) und zur ersten Öffnung (22) hin mündet.

3. Schalldetektor (1) nach einem der Ansprüche 1 oder 2, **dadurch gekennzeichnet, dass** das Mikrofon (13), oder das Mikrofon (13) und der Drucksensor (16), physikalische Eigenschaften erfassen, die in einer Akustikkammer (15) von konischer Form vorhanden sind, wobei der breitere Teil der ersten Öffnung (22) zugewandt ist.

4. Schalldetektor (1) nach einem der vorhergehenden Ansprüche, **dadurch gekennzeichnet, dass** er einen Positionssensor aufweist, und dadurch, dass der Schalldetektor Signale liefert, die für die Position des Detektors im Raum repräsentativ sind.

## Claims

1. Sound detector (1) comprising a housing (20) provided with a first opening (22) towards the outside for measuring static pressure and sound, said detector including a microphone (13) delivering signals representative of the sound entering via the first opening (22), said housing also including a pressure sensor (16) delivering signals representative of a static pressure and of a sound of a frequency lower than the frequencies captured by the microphone (13); where said microphone detects the sound entering through the first opening (22) through a membrane; **characterised in that** said membrane is impervious (12) and made of polyimide, and **in that** said membrane (12) is inserted between two substrate layers (11) of a printed circuit (10) supporting electronic circuits integrated into the sound detector (1).

2. Sound detector (1) according to claim 1, **characterised in that** the two substrate layers have a hole closed off by said membrane (12) and opening out towards the microphone (13) and towards the first opening (22).

3. Sound detector (1) according to any one of claims 1 or 2, **characterised in that** the microphone (13), or the microphone (13) and the pressure sensor (16), detect physical characteristics present in an acoustic chamber (15) that is conical in shape, the widest part being directed towards the first opening (22).

4. Sound detector (1) according to any one of the preceding claims, **characterised in that** it comprises a position sensor and **in that** said sound sensor delivers signals representative of the position of the detector in space.
